# EUROPÄISCHE PATENTSCHRIFT

(11) **EP 1 459 773 B1**
(45) Veröffentlichungstag und Bekanntmachung des Hinweises auf die Patenterteilung: **09.05.2012**
(21) Anmeldenummer: 04006294.5
(22) Anmeldetag: 17.03.2004
(51) Int. Cl.: A61M 1/36, G01N 33/49, B01L 3/00

(54) **Mikrostrukturierte Trennvorrichtung und mikrofluidisches Verfahren zum Abtrennen von flüssigen Bestandteilen aus einer Flüssigkeit, die Partikel enthält**
Microstructured separation device and microfluidic method for separating liquid components from a liquid containing particles
Dispositif de séparation à microstructure et méthode pour séparer des composants liquides d'un liquide contenant des particules

(30) Priorität: 21.03.2003 DE 10313201
(43) Veröffentlichungstag der Anmeldung: 22.09.2004
(73) Patentinhaber: Boehringer Ingelheim microParts GmbH, 44227 Dortmund (DE)
(72) Erfinder: Peters, Ralf-Peter, Dr. Dipl.-Phys., 51467 Bergisch-Gladbach (DE); Blankenstein, Gert, Dr., 44139 Dortmund (DE)
(74) Vertreter: Graefe, Jörg

(56) Entgegenhaltungen:
- EP-A- 0 269 240
- DE-A- 10 046 173
- DE-A- 10 150 549
- US-A- 5 922 210

## Beschreibung

Die Erfindung betrifft eine mikrostrukturierte Trennvorrichtung und ein mikrofluidisches Verfahren zum Abtrennen von flüssigen Bestandteilen aus einer Flüssigkeit, die Partikel enthält.

Eine derartige Vorrichtung wird beispielsweise benutzt, um Blutplasma von dem im Blut enthaltenden zellulären Bestandteilen (Hämatokrit) zu isolieren. Das Blut des Menschen setzt sich zum Einen aus dem flüssigen Blutplasma, dass etwa 55% des menschlichen Bluts ausmacht, und zum Anderen aus zellulären Bestandteilen, dem Hämatokrit zusammen, welcher etwa 45% des menschlichen Bluts umfassen, zusammen. Das Blutplasma ist eine gelbliche, wässerige Lösung von Proteinen, Kohlehydraten, Lipiden und Mineralsalzen welche auch Antikörper enthält. Das Blutplasma besteht zu 90% aus Wasser und zu 10% aus darin gelösten Stoffen. Das Hämatokrit besteht unter anderem aus roten Blutkörperchen (Erythrozyten) und weißen Blutkörperchen (Leukozyten). Die roten Blutkörperchen sind dabei die kleineren Partikel in dem Blut. Sie sind scheibenartig ausgebildet wobei sie einen Durchmesser von etwa 7,7 µm und eine Höhe von etwa 2 µm haben und leicht verformbar sind. Dagegen sind die weißen Blutkörperchen größer und sie bilden daher die größeren Partikel in dem Blut mit einer Größe von 7 bis 20 µm. Außerdem sind die weißen Blutkörperchen im Vergleich zu den roten Blutkörperchen nicht oder nur wenig verformbar.

Für viele medizinische Untersuchungen ist es notwendig, dass das Blutplasma von dem in dem Blut enthaltenen Hämatokrit isoliert wird, um das Blutplasma untersuchen zu können. Derzeit sind mehrere Vorrichtungen und Verfahren zum Isolieren von Blutplasma aus Blut bzw. zum Isolieren von flüssigen Bestandteilen einer größere und kleinere Partikel enthaltenden Flüssigkeit bekannt.

Ein bekanntes Verfahren zur Isolierung von Blutplasma aus Blut, ist die Sedimentation. Dazu wird chemisch aufbereitetes Blut in einen Behälter gefüllt und das Blut in dem Behälter solange gelagert, bis sich die zellulären Bestandteile des Bluts am Boden des Behälters absetzen und als Überstand das gelblich-klare Blutplasma zurückbleibt. Das Blutplasma kann zum Beispiel mittels einer Pipette abgeschöpft werden. Für die Sedimentation ist eine relativ lange Zeit notwendig, bis das Blutplasma gewonnen werden kann.

Bei einem weiteren Verfahren zur Isolierung von Blutplasma aus Blut wird eine Laborzentrifuge benutzt. Das Blut wird in die Laborzentrifuge gegeben, und das Blutplasma und die Partikel in dem Blut werden aufgrund der Zentrifugalkräfte voneinander getrennt. Für dieses Verfahren ist eine aufwendig gestaltete Laborzentrifuge erforderlich. Sowohl die Sedimentation als auch die Isolierung mittels einer Laborzentrifuge sind nur für relativ große Volumina von Blut geeignet.

Ein weiteres Verfahren ist die Filterung von Blut mittels eines mechanischen Filters mit einem offenporigen Filtermedium, dessen Durchtrittsöffnungen so bemessen sind, dass alle Partikel des Blutes zurückgehalten werden. So können weder die weißen Blutkörper noch die roten Blutkörper durch den Filter hindurch treten. Das Blut wird auf den Filter gegeben, wobei die Partikel durch den Filter zurückgehalten werden, während das Blutplasma in das Filtermedium eindringt und durch das Filtermedium durchsickert, sobald das Filtermedium mit Blutplasma vollständig beladen ist. Die Kapazität derartiger Filter ist begrenzt, da sich auf dem Filter ein Filterkuchen aufbaut, welcher den Filter verstopft. Derartige Filter sind bereits als Mikrostrukturen realisiert worden, wobei die gleichen Probleme hinsichtlich der Kapazität bzw. des Verstopfen des Filters auftreten wie bei größeren Filtern. Das abgetrennte und im Filtermedium enthaltene Plasma kann untersucht werden, ohne dass es aus dem Filtermedium herausgeholt wird, oder es kann durch zusätzliche Manipulation aus dem Filtermedium herausgeholt werden. Steht nur eine geringe Menge Blut zur Verfügung, wird dieses Verfahren mühsam.

Ferner ist ein Verfahren zur Isolierung von Blutplasma aus Blut bekannt, welches sich den Zweifach-Fung-Effekt zu Nutze macht. Bei der Vorrichtung nach Zweifach-Fung wird Blut mittels einer Pumpe durch einen Kanal transportiert, wobei der Transportkanal eine Verzweigungsstelle aufweist. Die Verzweigungsstelle bzw. die von der Verzweigungsstelle aus weiterführenden Kanäle sind so ausgelegt, dass der eine der weiterführenden Kanäle einen wesentlich größeren Anteil des Volumenstromes des herangeführten Bluts aufnimmt als der andere Kanal. Aufgrund des Zweifach-Fung-Effekts wird in dem Kanal, in welchem der größere Volumenstrom des Bluts transportiert wird, ein deutlich größerer Anteil der in dem Blut enthaltenden Partikel transportiert als in dem anderen Kanal. Durch eine Kaskadierung derartiger Verzweigungsstellen in dem Kanal mit dem jeweils kleineren Volumenstrom kann man dadurch die Isolierung des Plasmas von den Partikeln des Bluts erreichen. Eine derartige Vorrichtung erfordert einerseits einen aufwendigen Aufbau mit kaskadenartig angeordneten Verzweigungsstellen, und andererseits eine Pumpe, um den notwendigen Volumenstrom in den Transportkanälen zu erzeugen. Außerdem wird eine relativ große Flüssigkeitsmenge benötigt, damit eine ausreichende Menge der flüssigen Bestandteile isoliert werden kann. Kanäle, in denen der Zweifach-Fung-Effekt auftritt, sind in beiden Richtungen quer zur Strömungsrichtung größer als 10 µm; sie können zum Beispiel 25 µm tief und 50 µm breit sein.

Der Erfindung liegt die Aufgabe zu Grunde, eine Vorrichtung und ein Verfahren vorzuschlagen, bei welchem ohne Filtermedium und ohne Pumpe oder andere Hilfsmittel flüssige Bestandteile aus einer Flüssigkeit von in der Flüssigkeit enthaltenen Partikeln abgetrennt werden. Die Vorrichtung und das Verfahren sollen insbesondere für eine vorgelegte Flüssigkeitsmenge im Bereich einiger Mikroliter geeignet sein.

Diese Aufgabe wird erfindungsgemäß durch eine mikrostrukturierte Trennvorrichtung gemäß Anspruch 1 sowie durch ein mikrofluidisches Verfahren gemäß Anspruch 21 gelöst. Die erfindungsgemäße Vorrichtung weist einen Transportkanal zum Transport der vorgelegten - partikelhaltigen - Flüssigkeit sowie zumindest einen Trennbereich zum Abtrennen eines Teils der Flüssigkeit auf, wobei die abgetrennte Teilmenge partikelfrei oder partikelarm ist. Der Trennbereich weist eine Mikrostruktur auf, die so ausgestaltet ist, dass sie die größeren Partikel zurückhält und die kleineren Partikel im Trennbereich verzögert.

In einer ersten Ausführung der erfindungsgemäßen Vorrichtung kann der Transportkanal für die partikelhaltige Flüssigkeit ein Kanal sein, durch den eine unbegrenzte Menge der partikelhaltigen Flüssigkeit hindurchströmen kann. Weiter kann der Transport mit einem Einlass und einem Auslass für die partikelhaltige Flüssigkeit versehen sein. Bei dieser Ausführung wird eine begrenzte Menge der partikelhaltigen Flüssigkeit in den Einlass eingegeben. Der Trennbereich kann sich an einer Verzweigungsstelle des Transportkanals befinden. Die partikelhaltige Flüssigkeit wird am Eingang des Trennbereiches vorbeigeleitet, und eine Teilmenge der Flüssigkeit wird als Seitenstrom abgeleitet. An den Trennbereich kann sich ein Sammelraum für die abgetrennte Teilmenge der Flüssigkeit anschließen, der mit einer Entlüftungsöffnung versehen ist. Weiter kann der Trennbereich mit einem Ableitkanal für die abgetrennte Teilmenge der Flüssigkeit versehen sein.

In einer zweiten Ausführung der erfindungsgemäßen Vorrichtung kann sich der Trennbereich am Ende eines mit einem Einlass versehenen Transportkanals befinden. Bei dieser Ausführung wird die partikelhaltige Flüssigkeit dem Trennbereich direkt zugeleitet, und eine Teilmenge der Flüssigkeit wird über den Trennbereich weitergeleitet. An den Trennbereich kann sich ein Sammelraum für die abgetrennte Teilmenge der Flüssigkeit anschließen, der mit einer Entlüftungsöffnung versehen ist. Weiter kann der Trennbereich mit einem Ableitkanal für die abgetrennte Teilmenge der Flüssigkeit versehen sein. Bei dieser Ausführung liegen der Transportkanal, der Trennbereich und der Sammelraum hintereinander.

Die erfindungsgemäße Vorrichtung hat gegenüber den aus dem Stand der Technik bekannten Vorrichtungen mehrere Vorteile. Zum Einen kann die Flüssigkeit in dem Transportkanal ohne Pumpe transportiert werden. Für den Transport der Flüssigkeit in dem Transportkanal sind Kapillarkräfte ausreichend. Ein weiterer Vorteil ist, dass der Trennbereich neben dem Transportkanal angeordnet ist. Durch die in dem Transportkanal strömende Flüssigkeit können die Partikel der Flüssigkeit, die nicht in den Trennbereich eindringen, von dem Flüssigkeitsstrom in dem Transportkanal fortgespült werden. Vor dem Trennbereich bildet sich kein "Filterkuchen".

Die in den Trennbereich eintretende Flüssigkeit kann lediglich die kleineren Partikel enthalten. Diese werden beim Eintritt in den Trennbereich oder innerhalb des Trennbereiches gegenüber den flüssigen Bestandteilen der Flüssigkeit in ihrer Transportgeschwindigkeit abgebremst. Die flüssigen Bestandteile der Flüssigkeit werden schneller durch den Trennbereich transportiert als die kleineren Partikel. Dadurch können über einen längeren Zeitraum lediglich die flüssigen Bestandteile der Flüssigkeit zum Ende des Trennbereiches gelangen. Während dieses Zeitraums wird eine Menge der flüssigen Bestandteile durch den Trennbereich transportiert, die ausreichend für die gewünschten Analysen ist.

Das Abbremsen der kleineren Partikel beim Eintritt in den Trennbereich oder innerhalb des Trennbereiches wird durch die Mikrostruktur und durch Oberflächeneffekte, z.B. durch den "chromatographischen Effekt" erreicht. Wegen des "chromatographischen Effekts" können in einem gleichförmig ausgebildeten Kanal flüssige Bestandteile einer Flüssigkeit schneller transportiert werden als die darin enhaltenen Partikel. Dadurch können sich bei längeren Kanälen in der Transportrichtung zwei hintereinanderliegende Phasen ausbilden. Die erste Phase kann überwiegend oder nur flüssige Bestandteile enthalten, die zweite Phase kann sowohl flüssige Bestandteile als auch Partikel enthalten.

Gemäß der Erfindung kann die Mikrostruktur im Trennbereich eine oder mehrere Durchtrittsöffnungen begrenzen. Diese Durchtrittsöffnungen haben dabei eine Höhe, die geringer ist als die Höhe des Transportkanals. Die Durchtrittsöffnungen können z.B. von 0,5 bis 2 µm hoch sein. Beim Abtrennen von Blutplasma aus Vollblut können die roten Blutkörperchen aufgrund ihrer Verformbarkeit verzögert in den Trennbereich eindringen, während die weißen Blutkörperchen zu groß für die Durchtrittsöffnung sind. Mehrere Durchtrittsöffnungen können ganz oder teilweise nebeneinander angeordnet sein. Ferner können mehrere Durchtrittsöffnungen ganz oder teilweise im Seitenstrom hintereinander angeordnet sein. Die Breite der Durchtrittsöffnungen kann in Richtung des Seitenstromes beispielsweise von 10 µm auf 2 µm abnehmen. Ebenso kann die Höhe der Durchtrittsöffnungen im Seitenstrom abnehmen.

Gemäß der Erfindung kann die Länge des Trennbereiches kleiner sein als dessen Breite, wobei die Länge des Trennbereiches sich in der Richtung des Seitenstromes erstreckt. Beispielsweise kann die Länge 0,5 mm und die Breite 5 mm betragen. Eine erfindungsgemäße Trennvorrichtung kann eine oder mehrere Trennbereiche aufweisen, die hintereinander angeordnet sein können.

Die Mikrostruktur im Trennbereich kann eine Rampe, ein Spalt oder eine Treppe sein. Die Mikrostruktur kann beabstandete Säulen oder einen oder mehrere Stege umfassen. Die Spaltbreite kann in Richtung des Seitenstromes konstant sein, oder sie kann abnehmen oder zunehmen.

Ferner kann eine erfindungsgemäße Trennvorrichtung ein Sammelelement aufweisen, welches sich in Richtung des Seitenstromes an den Trennbereich anschließt. Dieses Sammelelement kann als Sammelkammer ausgebildet sein. Eine solche Sammelkammer kann zum Beispiel eine Grundfläche von 5 mm x 5 mm bei einer Höhe von 0,01 mm haben und ein Volumen von 0,25 Mikroliter. In diesem Sammelelement können Reagenzien vorgesehen sein. Diese Reagenzien können zu Analysezwecken mit den flüssigen Bestandteilen, welche in das Sammelelement eintreten, reagieren. Das Sammelelement kann über einen Entnahme- und/oder Entlüftungskanal mit der Umgebung verbunden sein. Die im Sammelelement enthaltene Flüssigkeit kann mittels einer Pumpe oder einer Spritze oder ähnlichem daraus entnommen werden. Über den Entlüftungskanal kann die im Trennbereich und im Sammelelement enthaltene Luft entweichen, sobald die Flüssigkeit in den Trennbereich und in das Sammelelement eintritt.

Gemäß der Erfindung kann die Trennvorrichtung einen Einlass aufweisen, welcher - in Richtung der Strömung im Transportkanal gesehen - vor dem Trennbereich liegt, und der mit dem Transportkanal verbunden ist. Die Trennvorrichtung kann einen Auslass aufweisen, der - in Transportrichtung gesehen - am Ende des Transportkanals liegt.

An den Trennbereich, das Sammelelement oder den Entlüftungskanal können sich andere mikrostrukturierte Elemente anschließen.

Ausführungsbeispiele für erfindungsgemäße Trennvorrichtungen sind anhand der Zeichnung näher beschrieben. Darin zeigt
- Fig. 1a: eine Draufsicht auf eine erfindungsgemäße Trennvorrichtung, in einer einfachen Ausführung,
- Fig. 1b: einen Schnitt durch die Trennvorrichtung gemäß Fig. 1a entlang der Linie Ib-Ib,
- Fig. 1c: einen Schnitt durch die erfindungsgemäße Trennvorrichtung gemäß Fig. 1 a entlang der Linie Ic-Ic,
- Fig. 2: einen Träger mit einer erfindungsgemäßen Trennvorrichtung,
- Fig. 3a: eine erfindungsgemäße Trennvorrichtung mit Kerben im Bereich des Übergangs zwischen einem Trennbereich und einer Sammelkammer,
- Fig. 3b: einen Schnitt durch die Trennvorrichtung gemäß Fig. 3a entlang der Linie IIIb-IIIb,
- Fig. 4: eine Trennvorrichtung mit einem zickzackförmigen Steg als Mikrostruktur,
- Fig. 5a: eine Trennvorrichtung mit Säulen im Trennbereich, die zusätzlich als Stützstruktur für ein Deckelelement dienen können,
- Fig. 5b: einen Schnitt durch die Trennvorrichtung gemäß Fig. 5a entlang der Linie Vb-Vb,
- Fig. 6: eine Trennvorrichtung mit versetzt zueinander angeordneten Säulen im Trennbereich,
- Fig. 7: eine Trennvorrichtung mit Säulen im Trennbereich, wobei die Abstände der Säulen zueinander in Strömungsrichtung abnehmen,
- Fig. 8: eine Trennvorrichtung mit einem gezackten Steg und Säulen im Trennbereich,
- Fig. 9a: eine Trennvorrichtung mit einer Rampe im Trennbereich,
- Fig. 9b: einen Schnitt durch die Trennvorrichtung gemäß Fig. 9a entlang der Linie IXb-IXb,
- Fig. 10a: eine Trennvorrichtung mit Treppenstufen im Trennbereich,
- Fig. 10b: einen Schnitt durch die Trennvorrichtung gemäß Fig. 10a entlang der Linie Xb-Xb,
- Fig. 11a: eine Trennvorrichtung mit einem ringförmig angeordneten Transportkanal, einem ringförmig angeordneten Trennbereich und einen innerhalb des Trennbereiches angeordneten Sammelraum,

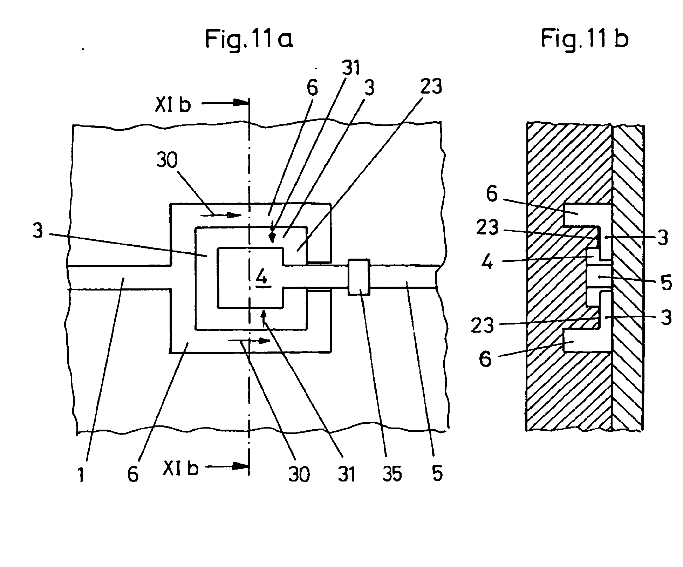

- Fig. 11 b: einen Schnitt durch die Trennvorrichtung gemäß Fig. 11a entlang der Linie Xlb-Xlb.

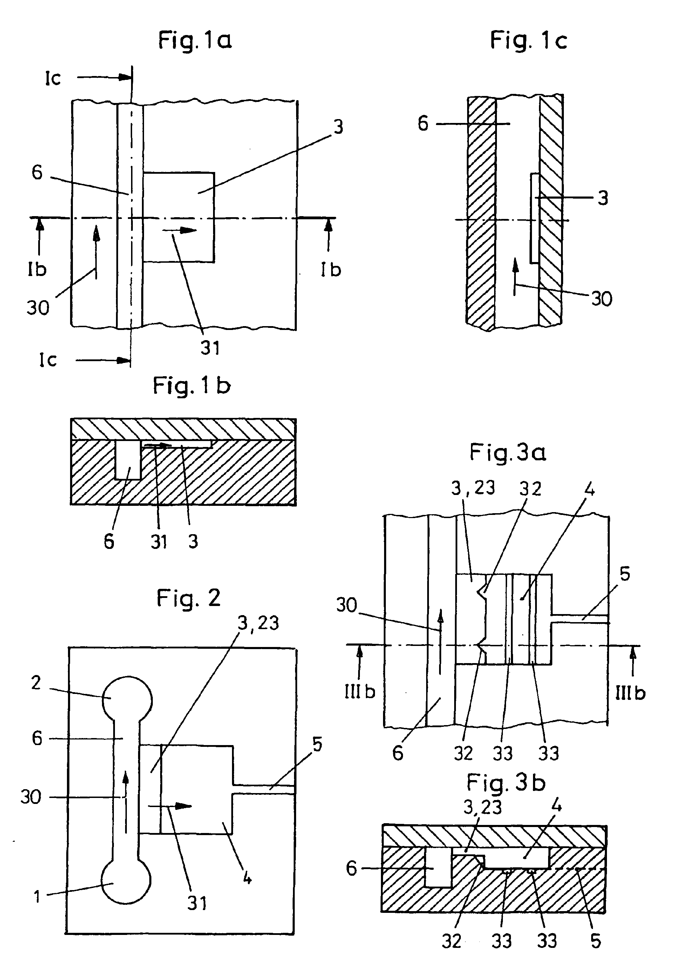

In den Figuren 1a bis 1c ist eine vereinfachte Trennvorrichtung dargestellt, anhand welcher das Funktionsprinzip einer erfindungsgemäßen Trennvorrichtung erläutert wird. Die in den Figuren dargestellten Trennvorrichtungen und somit auch die Trennvorrichtungen gemäß der Figuren 1a bis 1c sind so ausgelegt, dass mit ihnen zum Beispiel Blutplasma aus Vollblut abgetrennt werden kann. Mit einer erfindungsgemäßen Trennvorrichtung kann das im Vollblut enthaltene Blutplasma von dem im Blut enthaltenen Hämatokrit getrennt werden.

Die erfindungsgemäße Trennvorrichtung gemäß der Figuren 1a bis 1c weist dazu - wie auch die übrigen in den Figuren dargestellten Trennvorrichtungen - einen Transportkanal 6 und einen Trennbereich 3 auf. Das vorgelegte Blut wird in dem Transportkanal 6 in der Transportrichtung 30 transportiert. Das Blut kann allein durch Kapillarkräfte zwischen dem Anfang und dem Ende des Transportkanals 6 transportiert werden. Neben dem Transportkanal 6 ist ein Trennbereich 3 angeordnet. In diesem Trennbereich 3 verlangsamt sich die Fließgeschwindigkeit des Hämatokrit gegenüber der Fließgeschwindigkeit des Blutplasmas so, dass sich in der Transportrichtung 31 am Ende des Trennbereiches 3 von dem Hämatokrit isoliertes Blutplasma sammelt.

Sowohl der Transportkanal 6 als auch der Trennbereich 3 sind als Ausnehmung in der Oberfläche eines Trägers vorgesehen. Der Transportkanal 6 hat eine größere Tiefe als der Trennbereich 3. Der Übergang zwischen dem Transportkanal 6 und dem Trennbereich 3 wird daher durch einen Absatz gebildet. Die Ausnehmungen, d.h. der Transportkanal 6 und der Trennbereich 3, können mit einem Deckel abgedeckt sein, welcher beispielsweise aus dem gleichen Material wie der Träger oder aus einer Folie bestehen kann.

Der Absatz, der Deckel und die Seitenwände des Trennbereiches bilden die Mikrostruktur des Trennbereiches 3 mit einer definierten Durchtrittsöffnung. Diese Durchtrittsöffnung ist so bemessen, dass die größeren zellulären Bestandteile nicht durch die Durchtrittsöffnung gelangen können und von dem in dem Transportkanal 6 strömenden Blut fortgespült werden, und die Durchtrittsöffnung des Trennbereiches nicht verschließen können. Bei diesen Bestandteilen handelt es sich vorwiegend um die weißen Blutkörperchen. Die Durchtrittsöffnung ist vorteilhaft so bemessen, dass kleinere zelluläre Bestandteile nur dann die Durchtrittsöffnung passieren können, wenn sich diese kleineren zellulären Bestandteile verformen und an die Größe der Durchtrittsöffnung anpassen. Innerhalb des Trennbereiches 3 werden das Blutplasma und die kleineren Bestandteile des Bluts durch Kapillarkräfte transportiert. Die Kapillarkräfte im Trennbereich sind größer als die Kapillarkräfte im Transportkanal 6.

Ein weiterer Effekt, welcher sich bei einer erfindungsgemäßen Trennvorrichtung insbesondere zur Trennung des Blutplasmas von den kleineren zellulären Bestandteilen des Bluts auswirkt, ist der "chromatographische Effekt". Der chromatographische Effekt bewirkt, dass Blutplasma schneller durch den Trennbereich 3 transportiert wird als zelluläre Bestandteile, beispielsweise rote Blutkörperchen, die zwar in den Trennbereich 3 eindringen können, aber im Trennbereich 3 langsamer fortbewegt werden als das Blutplasma. Bevor die zellulären Bestandteile den Sammelbereich am Ende des Trennbereiches 3 erreichen können, ist dieser schon vollständig mit Blutplasma gefüllt. Die roten Blutkörperchen können nicht mehr in den Sammelbereich eindringen und in das Blutplasma gelangen.

In Fig. 2 ist ein Träger mit einer Trennvorrichtung dargestellt, bei der ein Einlass 1 und ein Auslass 2 vorgesehen sind, die über den Transportkanal 6 miteinander verbunden sind. An den Transportkanal 6 schließt sich ein Trennbereich 3 an. Dieser Trennbereich 3 wird in Richtung 31 von dem abzutrennenden Blutplasma durchströmt. In der Richtung 31 schließt sich an den Trennbereich 3 ein als Sammelkammer 4 ausgebildetes Sammelelement an. Diese Sammelkammer 4 ist über einen Entnahme- und Entlüftungskanal 5 mit der Umgebung verbunden.

An das Ende des Entlüftungs- und Entnahmekanals kann eine Spritze oder eine Pumpe angeschlossen werden, um das abgetrennte Blutplasma aus der Sammelkammer zu entnehmen. Über den Entnahme- und Entlüftungskanal wird die in der Sammelkammer 4 und im Trennbereich 3 enthaltene Luft, die beim Eintritt des Blutplasmas in die Sammelkammer 4 und in den Trennbereich 3 verdrängt wird, abgeführt. Ebenso kann der Sammelbereich und die Sammelkammer durch eine Öffnung im Deckel entlüftet werden.

Der Einlass 1, der Auslass 2, der Transportkanal 6, der Trennbereich 3, die Sammelkammer 4 und der Entnahme- und Entlüftungskanal 5 sind als Ausnehmungen in dem Träger vorgesehen. Dabei sind der Einlass, der Transportkanal 6 und der Auslass 2 so ausgestaltet, dass in den Einlass 1 gegebenes Blut auf Grund der in dem Einlass 1, dem Transportkanal 6 und dem Auslass 2 wirkenden Kapillarkräfte vom Einlass 1 über den Transportkanal 6 zum Auslass 2 transportiert wird.

Im Trennbereich 3 wirken Kapillarkräfte, welche größer sind als die Kapillarkräfte, im Transportkanal 6. Dadurch wird ein Teil des in dem Transportkanal fließenden Bluts in den Trennbereich 3 abgezweigt. Der Boden des Trägers im Trennbereich 3, die Seitenwände der Ausnehmung sowie der Deckel auf dem Träger bilden eine Durchtrittsöffnung, deren Höhe geringer ist als die Höhe des Transportkanals 6. Da die Höhe der Sammelkammer 4 größer ist als die Höhe der Durchtrittsöffnung des Trennbereiches 3, ist die Mikrostruktur des Trennbereiches 3 als Steg 23 ausgebildet. Die Höhe der Durchtrittsöffnung zwischen Steg 23 und Deckel ist so bemessen, dass insbesondere die größeren zellulären Bestandteile des Bluts nicht zwischen dem Steg 23 des Trennbereiches 3 und dem Deckel der Trennvorrichtung hindurch treten können. Es kann lediglich das Blutplasma und je nach Höhe der Durchtrittsöffnung gegebenenfalls kleinere zelluläre Bestandteile des Bluts auf Grund der im Trennbereich 3 wirkenden Kapillarkräfte von dem Transportkanal 6 in die Sammelkammer 4 transportiert werden. Die sich vor der Eingangsöffnung des Trennbereiches 3 sammelnden zellulären Bestandteile des Blutes werden nicht abgelagert, wodurch der Trennbereich 3 verstopfen könnte, sondern werden durch das aus dem Einlass nachströmende Blut in der Transportrichtung 30 zum Auslass 2 transportiert, wo gegebenenfalls Blut mit einer erhöhten Konzentration von Hämatokrit gesammelt wird.

Im Trennbereich 3 wird auf Grund des "chromatographischen Effekts" das Blutplasma von den gegebenenfalls noch in der Flüssigkeit enthaltenen kleineren zellulären Bestandteilen getrennt.

Anhand der Figuren 3a bis 10b werden verschiedene Trennvorrichtungen mit einem Transportkanal 6, einem Trennbereich 3 und einer Sammelkammer 4 sowie einem Entnahme- und Entlüftungskanal 5 beschrieben, wie sie in einem Träger gemäß Fig. 2 oder in anderen Trägern entsprechend eingesetzt werden können. Die Trennvorrichtungen der Figuren 3a bis 10b unterscheiden sich von der bei dem Träger gemäß Fig. 2 verwendeten Trennvorrichtung im Wesentlichen durch die Ausgestaltung des Trennbereiches 3 oder durch die Ausgestaltung der Sammelkammer 4.

Bei der Trennvorrichtung gemäß den Figuren 3a und 3b sind im Unterschied zum Trennbereich 3 gemäß Fig. 2 im Übergang vom Trennbereich 3 zur Sammelkammer 4 zwei Kerben 32 in der Seitenfläche des Steges. Diese Kerben verbinden, wie aus der Schnittdarstellung der Figur 3b ersichtlich ist, den Trennbereich 3 mit dem Boden der Sammelkammer 4. Diese Kerben überwinden den Kapillarstopp der sich gegebenenfalls durch die schlagartige Änderung der geometrischen Eigenschaften im Bereich des Übergangs vom Trennbereich 3 zur Sammelkammer 4 ausbildet. Das über den Trennbereich 3 fließende Blutplasma wird in den Kerben transportiert.

Ein weiterer Unterschied zwischen der Trennvorrichtung gemäß Fig. 2 und der Trennvorrichtung gemäß Fig. 3a und 3b ist, dass bei der Trennvorrichtung gemäß Fig. 3a und 3b in dem Boden der Sammelkammer 4 quer zur Transportrichtung 31 liegende Nuten 33 vorgesehen sind. Diese Nuten vergleichmäßigen und begradigen eine herannahende Flüssigkeitsfront aus Blutplasma. Durch die Nuten 33 wird die Flüssigkeitsfront zunächst aufgehalten, bis der gesamte Bereich zwischen der Nut 33 und dem Trennbereich 3 mit Blutplasma ausgefüllt ist. Das nachströmende Blutplasma drückt das Blutplasma über die Nut 33 hinweg. Die Sammelkammer 4 wird dadurch gleichmäßig mit Blutplasma aufgefüllt. Die zunächst in der Sammelkammer 4 enthaltene Luft wird ohne die Bildung von Luftblasen über den Entnahme- und Entlüftungskanal 5 aus der Sammelkammer 4 herausgeführt.

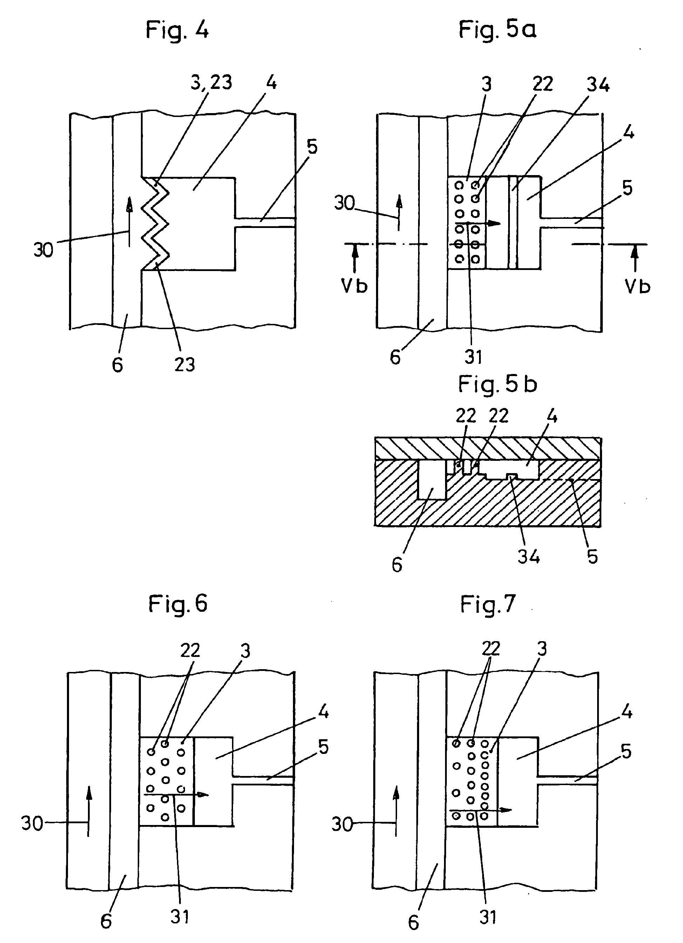

Das in Fig. 4 dargestellte Ausführungsbeispiel für eine erfindungsgemäße Trennvorrichtung unterscheidet sich von der Trennvorrichtung gemäß Fig. 2 dadurch, dass anstelle eines geraden Steges 23 mit parallel zu der Transportrichtung 30 liegenden Kanten ein gezackter Steg 23' verwendet wird. Durch die Zackenform des Steges 23' wird die Kontaktfläche des Steges 23' und die effektive Kontaktfläche im Trennbereich 3 vergrößert.

Bei den in den Figuren 5a bis 7 verwendeten Trennvorrichtungen ist jeweils ein Steg vorgesehen, bei dem sich zwischen der Oberfläche des Steges und der Unterseite des Deckels (in Fig. 5a, Fig. 6 und Fig. 7 nicht dargestellt) Säulen 22 erstrecken. Wegen der Säulen 22 weist der Trennbereich 3 nicht nur eine Durchtrittsöffnung auf, sondern der Trennbereich 3 wird durch die Säulen 22 in mehrere Durchtrittsöffnungen unterteilt.

Die Säulen 22 sind bei der Trennvorrichtung gemäß der Figuren 5a und 5b in zwei Reihen mit hintereinander liegenden Säulen angeordnet. Die Durchtrittsöffnungen, welche durch die Säulen der ersten Reihe begrenzt werden, können die gleiche Breite haben wie die Durchtrittsöffnungen, welche durch die Säulen der zweiten Reihe begrenzt werden. Die Trennvorrichtung gemäß Fig. 5a und 5b weist in der Sammelkammer 4 einen quer zur Transportrichtung 31 liegenden Steg 34 auf. Der Steg 34 staut die davor anstehende Flüssigkeit zunächst auf. Sobald der Bereich vor dem Steg 34 vollständig gefüllt ist, überwindet die Flüssigkeit den Steg 34 und dringt in den hinter dem Steg 34 liegenden Bereich der Sammelkammer 4 ein. Dadurch wird ähnlich wie durch die Nuten 33 erreicht, dass das in die Sammelkammer 4 eindringende Blutplasma gleichförmig die Sammelkammer 4 befüllt und die in der Sammelkammer 4 enthaltene Luft ohne die Bildung von Luftblasen in der Sammelkammer 4 durch den Entlüftungs- und Entnahmekanal 5 austritt.

Im Gegensatz zu der Trennvorrichtung gemäß Fig. 5a und 5b sind bei einer Trennvorrichtung gemäß Fig. 6 die Säulen 22 in drei Reihen versetzt hintereinander angeordnet, d.h. die Säulen der zweiten Reihe fluchten mit den Durchtrittsöffnungen zwischen den Säulen der ersten Reihe und die Säulen der dritten Reihe fluchten mit den Durchtrittsöffnungen zwischen den Säulen der zweiten Reihe. Durch diese versetze Anordnung der Säulen 22 werden in der Flüssigkeit mitgeführte kleinere zelluläre Bestandteile durch Anstoßen an den Säulen abgebremst, wodurch das schneller strömende Blutplasma mehr Zeit hat, die Sammelkammer 4 zu befüllen, bevor der erste zelluläre Bestandteil die Sammelkammer 4 erreicht.

Bei der Trennvorrichtung gemäß Fig. 7 haben die Durchtrittsöffnungen zwischen den Säulen der ersten Reihe eine größere Breite als die Durchtrittsöffnungen zwischen den Säulen der zweiten Reihe. Die Durchtrittsöffnung zwischen den Säulen der zweiten Reihe haben eine größere Breite als die Durchtrittsöffnungen zwischen den Säulen der dritten Reihe. Eine derartige Mikrostruktur hat den Vorteil, dass zelluläre Bestandteile, welche vom Transportkanal 6 in den Trennbereich 3 gegebenenfalls eingedrungen sind, je nach Größe in der ersten, zweiten oder dritten Reihe der Säulen 22 aufgehalten werden, ohne dass der Trennbereich verstopft.

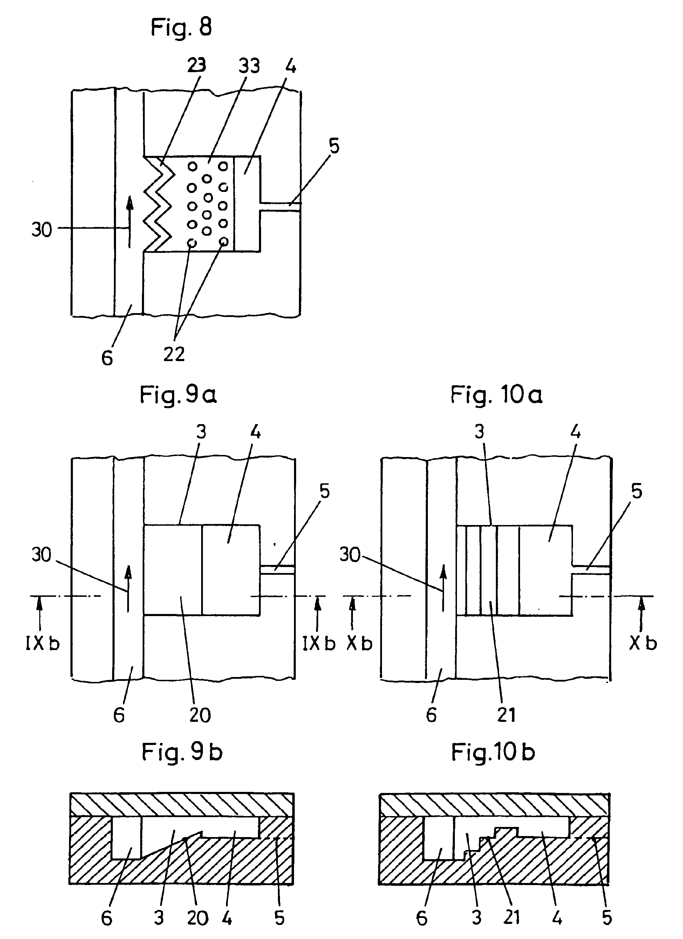

Die Trennvorrichtung gemäß Fig. 8 weist als Mikrostrukturen sowohl einen Steg 23 als auch Säulen 22 auf. Der Steg 23 ist in dem an den Transportkanal 6 angrenzenden Bereich des Trennbereiches 3 vorgesehen und erstreckt sich zickzackförmig neben dem Transportkanal 6. Hinter dem Steg 23 sind in drei Reihen gestaffelt und auf Lücke gesetzt Säulen 22 angeordnet.

Die Höhe der Durchtrittsöffnung im Bereich des Steges 23 ist dabei so ausgestaltet, dass kleinere zelluläre Bestandteile des Blutes, wie zum Beispiel rote Blutkörperchen, durch den Zwischenraum zwischen dem Steg 23 und dem Deckel hindurch treten können, diese Bestandteile jedoch von den Säulen 22 aufgehalten werden.

In Fig. 9a und 9b ist eine Trennvorrichtung dargestellt, bei welcher der Trennbereich 3 durch eine Rampe 20 gebildet wird. Diese Rampe 20 steigt von dem Niveau des Bodens des Transportkanals 6 an. Durch die Durchtrittsöffnung am Ende der Rampe können die kleinsten zellulären Bestandteile nicht hindurch treten. Der vordere Bereich der Rampe 20, welcher sich unmittelbar an den Transportkanal 6 anschließt, wird durch das in dem Transportkanal fließende Blut ständig gespült. In diesem Bereich vorhandene Partikel werden vom Flüssigkeitsstrom im Transportkanal 6 weggespült.

Die Trennvorrichtung gemäß der Figuren 10a und 10b weist einen Trennbereich 3 mit einer Mikrostruktur auf, welche durch eine Treppe 21 gebildet wird. Durch die Treppe 21 wird die Höhe zwischen der Treppe 21 und dem Deckel schrittweise herabgesetzt. Durch die Durchtrittsöffnung zwischen der letzten Stufe und dem Deckel können die kleinsten zellulären Bestandteile des Blutes, d.h. insbesondere die roten Blutkörperchen, nicht oder nur verzögert hindurchtreten.

In den Figuren 11a und 11b ist eine weitere Ausführung einer erfindungsgemäßen Trennvorrichtung dargestellt. Diese Trennvorrichtung weist als Einlass 1 einen Kanal auf, an welchen sich ein in der Draufsicht C-förmiger Transportkanal 6 anschließt. Der Transportkanal 6 ist dabei im Bereich des die beiden Schenkel des "C" verbindenden Stegs angeschlossen. Der Transportkanal 6 hat zwei Transportkanalhälften, welche mit dem Ende des Einlasses 1 verbunden sind. Entlang der Innenseite beider Hälften des Transportkanals 6 erstreckt sich, ebenfalls in Draufsicht C-förmig, ein Trennbereich 3. Der Innenraum dieses Trennbereiches 3 ist die Sammelkammer 4, welche mit einem Entnahme- und Entlüftungskanal 5 verbunden ist, der durch die offene Seite des in Draufsicht C-förmigen Trennbereiches 3 und des in Draufsicht C-förmigen Transportkanals 6 hindurch geführt ist. Sowohl der Einlass 1 als auch der Transportkanal 6 beziehungsweise die Transportkanalhälften sind so ausgebildet, dass das über den Einlass in die Trennvorrichtung eingebrachte Blut auf Grund der in dem Einlass 1 und den Hälften des Transportkanals 6 wirkenden Kapillarkräfte vom Ende des Einlasses 1 zu den Enden der Hälften des Transportkanals 6 transportiert wird.

Die Enden der Transportkanalhälften sind z.B. mit einem Auslass verbunden, welcher das überschüssige Blut aus den Transportkanalhälften aufnimmt.

Der Trennbereich 3 weist eine Mikrostruktur auf, welche durch einen Steg 23 gebildet wird, der sich zwischen den Transportkanalhälften 6 und der Sammelkammer 4 erstreckt. Zwischen dem Steg 23 und einem Deckel der erfindungsgemäßen Trennvorrichtung, welcher die Kanäle 5, 6, den Trennbereich 3 und die Sammelkammer 4 abdeckt, verbleibt eine Durchtrittsöffnung, die so hoch ist, dass die größeren zellulären Bestandteile nicht durch diese hindurchdringen können. Der Steg hat in Transportrichtung 31 eine Ausdehnung, welche so bemessen ist, dass kleinere zelluläre Bestandteile, wie beispielsweise rote Blutkörperchen erst dann an den inneren Rand des Steges 23 gelangen, wenn die Sammelkammer 4 bereits vollständig mit Blutplasma gefüllt ist. Sobald die Sammelkammer 4 vollständig gefüllt ist, können die roten Blutkörperchen, welche sich im Trennbereich 3 befinden, auf Grund der stoppenden Transportmechanismen nicht in die Sammelkammer 4 transportiert werden, so dass eine Vermischung des Blutplasmas mit roten Blutkörperchen verhindert wird.

In der dargestellten Ausführung umfasst der Entnahme- und Entlüftungskanal 5 einen Kapillarstopp 35, welcher durch das Anlegen eines äußeren Drucks, z.B. durch eine Spritze oder durch eine Pumpe, überwunden werden kann, um das abgetrennte Blutplasma aus der Sammelkammer zu entnehmen.

## Patentansprüche

1. Mikrostrukturierte Trennvorrichtung zum Abtrennen von flüssigen Bestandteilen aus einer Flüssigkeit, die Partikel enthält, umfassend:
- zumindest einen Transportkanal (6) zum Transport der Flüssigkeit in eine vorgegebene Transportrichtung (30);
- zumindest einen Trennbereich (3) an einer Verzweigungsstelle des Transportkanals, an die sich ein Seitenkanal anschließt, durch den ein Teilstrom der Flüssigkeit aus dem Transportkanal abgeleitet wird; sowie
- eine Mikrostruktur (20, 21, 22, 23) im Trennbereich (3), die größere Partikel der Flüssigkeit aus dem Trennbereich heraushält und die den Transport kleinerer Partikel im Trennbereich (3) verzögert.

2. Trennvorrichtung nach dem vorhergehenden Anspruch, **dadurch gekennzeichnet, dass** die Mikrostrukturen (20, 21, 22, 23) eine oder mehrere Durchtrittsöffnungen begrenzen.

3. Trennvorrichtung nach dem vorhergehenden Anspruch, **dadurch gekennzeichnet, dass** die Durchtrittsöffnungen eine Höhe haben die geringer sind als die Höhe des Transportkanals (6).

4. Trennvorrichtung nach Anspruch 2 oder 3, **dadurch gekennzeichnet, dass** die Höhe der Durchtrittsöffnungen 0,5 bis 1000 µm beträgt.

5. Trennvorrichtung nach einem der Ansprüche 2 bis 4, **dadurch gekennzeichnet, dass** die Durchtrittsöffnungen ganz oder teilweise nebeneinander angeordnet sind.

6. Trennvorrichtung nach einem der Ansprüche 2 bis 5, **dadurch gekennzeichnet, dass** die Durchtrittsöffnungen ganz oder teilweise in einer Transportrichtung (31 ) hintereinander angeordnet sind.

7. Trennvorrichtung nach dem vorhergehenden Anspruch, **dadurch gekennzeichnet, dass** die Breite der Durchtrittsöffnungen in Transportrichtung (31) abnimmt.

8. Trennvorrichtung nach Anspruch 6 oder 7, **dadurch gekennzeichnet, dass** die Höhe der Durchtrittsöffnungen in Transportrichtung (31) abnimmt.

9. Trennvorrichtung nach einem der vorhergehenden Ansprüche, **dadurch gekennzeichnet, dass** der Trennbereich (3) eine oder mehrere Mikrostrukturen (20, 21, 22, 23, 24) aufweist.

10. Trennvorrichtung nach dem vorhergehenden Anspruch, **dadurch gekennzeichnet, dass** die Mikrostruktur eine Rampe (20) ist.

11. Trennvorrichtung nach Anspruch 9 oder 10, **dadurch gekennzeichnet, dass** die Mikrostruktur eine Treppe (21) ist.

12. Trennvorrichtung nach einem der Ansprüche 9 bis 11, **dadurch gekennzeichnet, dass** die Mikrostruktur voneinander beabstandete Säulen (22) umfasst.

13. Trennvorrichtung nach einem der Ansprüche 9 bis 12, **dadurch gekennzeichnet, dass** die Mikrostruktur einen oder mehrere Stege (23) umfasst.

14. Trennvorrichtung nach einem der vorhergehenden Ansprüche, **dadurch gekennzeichnet, dass** sich in Transportrichtung (31) an den Trennbereich ein Sammelelement (4) anschließt.

15. Trennvorrichtung nach dem vorhergehenden Anspruch, **dadurch gekennzeichnet, dass** das Sammelelement (4) als Sammelkammer ausgebildet ist.

16. Trennvorrichtung nach Anspruch 14 oder 15, **dadurch gekennzeichnet, dass** das Sammelelement (4) Reagenzien enthält.

17. Trennvorrichtung nach dem vorhergehenden Anspruch, **dadurch gekennzeichnet, dass** sich an das Sammelelement ein Entnahme- und/ oder Entlüftungskanal (5) anschließt.

18. Trennvorrichtung nach dem Anspruch 17, **dadurch gekennzeichnet, dass** sich an den Trennbereich (3) ein Entnahme- und/ oder Entlüftungskanal (5) anschließt.

19. Trennvorrichtung nach einem der vorhergehenden Ansprüche, **dadurch gekennzeichnet, dass** die Trennvorrichtung einen Einlass (1) aufweist.

20. Trennvorrichtung nach einem der vorhergehenden Ansprüche, **dadurch gekennzeichnet, dass** die Trennvorrichtung einen Auslass (2) aufweist, der in Transportrichtung am Ende des Transportkanals (6) anschließt.

21. Mikrofluidisches Verfahren zum Abtrennen von flüssigen Bestandteilen aus einer Flüssigkeit, die Partikel enthält, **gekennzeichnet durch**:
- die Flüssigkeit wird in einem Transportkanal (6) transportiert;
- ein Teil der Flüssigkeit wird über einen Trennbereich (3) aus dem Transportkanal abgezweigt;
- wobei größere Partikel beim Eintritt in den Trennbereich (3) zurückgehalten und von der im Transportkanal strömenden Flüssigkeit fortgespült werden und
- wobei kleinere Partikel beim Eintritt in den Trennbereich (3) oder beim Transport in den Trennbereich (3) abgebremst werden.

## Claims

1. Microstructured separation device for separating liquid components from a liquid which contains particles, comprising:
- at least one transport channel (6) for transporting the liquid in a pre-specified transport direction (30);
- at least one separation region (3) at a branching location of the transport channel, which separation region (3) is connected to a side channel, through which a particle stream of the liquid is diverted from the transport channel; and
- a microstructure (20, 21, 22, 23) in the separation region (3), which keeps larger particles of the liquid out of the separation region and slows the transport of smaller particles in the separation region (3).

2. Separation device according to the preceding claim, **characterized in that** the microstructures (20, 21, 22, 23) delimit one or more openings.

3. Separation device according to the preceding claim, **characterized in that** the openings have a height that is less than the height of the transport channel (6).

4. Separation device according to Claim 2 or 3, **characterized in that** the height of the openings is 0.5 to 1000 µm.

5. Separation device according to one of Claims 2 to 4, **characterized in that** the openings are arranged entirely or partially next to one another.

6. Separation device according to one of Claims 2 to 5, **characterized in that** the openings are arranged entirely or partially one behind the other in a transport direction (31).

7. Separation device according to the preceding claim, **characterized in that** the width of the openings decreases in transport direction (31).

8. Separation device according to Claim 6 or 7, **characterized in that** the height of the openings decreases in transport direction (31).

9. Separation device according to one of the preceding claims, **characterized in that** the separation region (3) has one or more microstructures (20, 21, 22, 23, 24).

10. Separation device according to the preceding claim, **characterized in that** the microstructure is a ramp (20).

11. Separation device according to Claim 9 or 10, **characterized in that** the microstructure is a series of steps (21).

12. Separation device according to one of Claims 9 to 11, **characterized in that** the microstructure comprises mutually spaced apart columns (22).

13. Separation device according to one of Claims 9 to 12, **characterized in that** the microstructure comprises one or more bars (23).

14. Separation device according to one of the preceding claims, **characterized in that** a collection element (4) is connected to the separation region in transport direction (31).

15. Separation device according to the preceding claim, **characterized in that** the collection element (4) is configured as a collection chamber.

16. Separation device according to Claim 14 or 15, **characterized in that** the collection element (4) contains reagents.

17. Separation device according to the preceding claim, **characterized in that** a removal and/or ventilation channel (5) is connected to the collection element.

18. Separation device according to Claim 17, **characterized in that** a removal and/or ventilation channel (5) is connected to the separation region (3).

19. Separation device according to one of the preceding claims, **characterized in that** the separation apparatus has an inlet (1).

20. Separation device according to one of the preceding claims, **characterized in that** the separation apparatus has an outlet (2), which is connected to the end of the transport channel (6) in transport direction.

21. Microfluidic method for separating liquid components from a liquid, which contains particles, **characterized by**:
- the liquid being transported in a transport channel (6);
- part of the liquid being branched off from the transport channel via a separation region (3);
- wherein larger particles are kept back as they enter the separation region (3) and are flushed away by the liquid flowing in the transport channel and
- wherein smaller particles are decelerated as they enter the separation region (3) or as they are transported into the separation region (3).

## Revendications

1. Dispositif de séparation microstructuré destiné à séparer des composants liquides d'un liquide qui contient des particules, le dispositif comprenant :
au moins un canal de transport (6) qui transporte le liquide dans une direction prédéterminée de transport (30),
au moins une zone de séparation (3) située en une bifurcation du canal de transport à laquelle se raccorde un canal latéral par lequel une partie de l'écoulement de liquide est extraite du canal de transport et
une microstructure (20, 21, 22, 23) prévue dans la zone de séparation (3), qui extrait hors de la zone de séparation les particules grossières du liquide et qui ralentit le transport des particules fines dans la zone de séparation (3).

2. Dispositif de séparation selon la revendication précédente, **caractérisé en ce que** les microstructures (20, 21, 22, 23) délimitent une ou plusieurs ouvertures de passage.

3. Dispositif de séparation selon la revendication précédente, **caractérisé en ce que** la hauteur des ouvertures de passage est inférieure à la hauteur du canal de transport (6).

4. Dispositif de séparation selon les revendications 2 ou 3, **caractérisé en ce que** la hauteur des ouvertures de passage est de 0,5 à 1 000 µm.

5. Dispositif de séparation selon l'une des revendications 2 à 4, **caractérisé en ce que** la totalité ou une partie des ouvertures sont disposées les unes à côté des autres.

6. Dispositif de séparation selon l'une des revendications 2 à 5, **caractérisé en ce que** la totalité ou une partie des ouvertures sont disposées les unes à la suite des autres dans une direction de transport (31).

7. Dispositif de séparation selon l'une des revendications précédentes, **caractérisé en ce que** la largeur des ouvertures de passage diminue dans la direction de transport (31).

8. Dispositif de séparation selon les revendications 6 ou 7, **caractérisé en ce que** la hauteur des ouvertures de passage diminue dans la direction de transport (31).

9. Dispositif de séparation selon l'une des revendications précédentes, **caractérisé en ce que** la zone de séparation (3) présente une ou plusieurs microstructures (20, 21, 22, 23, 24).

10. Dispositif de séparation selon la revendication précédente, **caractérisé en ce que** la microstructure est une rampe (20).

11. Dispositif de séparation selon les revendications 9 ou 10, **caractérisé en ce que** la microstructure est un gradin (21).

12. Dispositif de séparation selon l'une des revendications 9 à 11, **caractérisé en ce que** la microstructure comporte des colonnes (22) disposées à distance les unes des autres.

13. Dispositif de séparation selon l'une des revendications 9 à 12, **caractérisé en ce que** la microstructure comporte une ou plusieurs nervures (23).

14. Dispositif de séparation selon l'une des revendications précédentes, **caractérisé en ce qu'**un élément de collecte (4) se raccorde à la zone de séparation dans la direction de transport (31).

15. Dispositif de séparation selon la revendication précédente, **caractérisé en ce que** l'élément de collecte (4) est configuré comme chambre de collecte.

16. Dispositif de séparation selon les revendications 14 ou 15, **caractérisé en ce que** l'élément de collecte (4) contient des réactifs.

17. Dispositif de séparation selon la revendication précédente, **caractérisé en ce qu'**un canal de prélèvement et/ou d'évent (5) se raccorde à l'élément de collecte.

18. Dispositif de séparation selon la revendication 17, **caractérisé en ce qu'**un canal de prélèvement et/ou d'évent (5) se raccorde à la zone de séparation (3).

19. Dispositif de séparation selon l'une des revendications précédentes, **caractérisé en ce que** le dispositif de séparation présente une entrée (1).

20. Dispositif de séparation selon l'une des revendications précédentes, **caractérisé en ce que** le dispositif de séparation présente une sortie (2) adjacente à l'extrémité du canal de transport (6) dans la direction de transport.

21. Procédé dit microfluide de séparation de composants liquides d'un liquide qui contient des particules, le procédé étant **caractérisé par** les étapes suivantes :
le liquide est transporté dans un canal de transport (6),
une partie du liquide est extraite du canal de transport dans une zone de séparation (3),
les particules qui entrent dans la zone de séparation (3) étant retenues et séparées du liquide qui s'écoule dans le canal de transport et
les petites particules sont freinées lorsqu'elles pénètrent dans la zone de séparation (3) ou lorsqu'elles pénètrent dans la zone de séparation (3).
